# EUROPEAN PATENT APPLICATION

(11) **EP 3 578 973 A1**
(43) Date of publication of application: **11.12.2019**
(21) Application number: 19179017.9
(22) Date of filing: 07.06.2019
(51) Int. Cl.: G01N 33/49

(54) **A METHOD FOR EVALUATION OF THE OXIDATIVE STRESS IN BIOLOGICAL SAMPLES AND DEVICE FOR ACHIEVING SUCH METHOD**

(30) Priority: 08.06.2018 IT 201800006166
(71) Applicant: Universita' Degli Studi di Firenze, 50121 Firenze (IT); Università degli Studi di Siena, 53100 Siena (IT); Università degli Studi Guglielmo Marconi - Telematica, 00193 Roma (IT)
(72) Inventor: FORT, Ada, 50141 Firenze (IT); VIGNOLI, Valerio, 50137 Firenze (IT); FIORILLO, Claudia, 50131 Firenze (IT); BECATTI, Matteo, 58036 Sticciano Scalo GR (IT); TADDEI, Niccolò, 50142 Firenze (IT); LOTTI, Torello, 50129 Firenze (IT); BARYGINA, Victoria, 50129 Firenze (IT); MUGNAINI, Marco, 50132 Firenze (IT); TANI, Marco, 53036 Poggibonsi SI (IT)
(74) Representative: Brazzini, Silvia

(57) **Abstract**

The present invention refers to a method for evaluation of the oxidative stress status in a subject based on the measurement of impedance in a sample of a biological fluid of the same subject, and to a device for achieving this method.

## Description

### Field of the Invention

The present invention is in the field of biomedical analyses, and it relates in particular to a method for the evaluation of oxidative stress in a biological sample of a patient, and to a device for achieving such a method.

### State of the Art

The reactive oxygen species (in the following also ROS, an acronym derived from the English expression Reactive Oxygen Species) are reactive chemical species containing oxygen that may or may not comprise unpaired electrons.

The ROS species have a short half-life; they are produced by aerobic cells during their metabolic processes (for example in mitochondrial respiration) and they react quickly by extracting electrons from other molecules, oxidizing them, forming very reactive compounds, often more reactive than the starting species that have formed them. Therefore, due to their nature and to these reactions in which they are involved, the ROS species damage all the main biological macromolecules (lipids, proteins, DNA, carbohydrates), thus influencing the redox state of cells, tissues and body fluids. Examples of ROS are peroxides, superoxides, hydroxyl radicals, and singlet oxygen.

Under physiological conditions, the ROS species are present at the cellular level where they are in equilibrium with antioxidant molecules. When this equilibrium is lacking due to the lack of antioxidant molecules and / or due to an excessive accumulation of ROS species, a condition called "oxidative stress" can occur. Oxidative stress is a condition present in numerous pathological conditions (from acute diseases such as myocardial infarction to chronic diseases such as Alzheimer's disease, atherosclerosis, tumours) and physiological conditions (exercise, aging, nutrition). A common characteristic of many diseases, in fact, is the production and accumulation of ROS, which also occurs on the occasion of environmental damages. This accumulation of ROS can induce severe cell damages and physiological dysfunction up to cell death.

Without reaching such extreme conditions, however, a limited oxidative damage can take place even under normal conditions, although the mechanisms behind it are still poorly understood. It is increasing the number of evidences suggesting how oxidative damage can be caused by ROS species as one of the first determining causes of aging and shortening of life span.

Such knowledge on the role of the ROS species in pathological processes has led research laboratories and clinical researchers to increasingly in-depth studies in order to understand better the chemistry of damages induced by oxidizing species, and to be able to use the information thus obtained to design new strategies and better protect the organism against pathophysiological consequences caused by oxidative stress.

So far, we know that, in case of oxidative stress, the cell activates defense mechanisms such as activation or silencing of genes that code for antioxidant enzymes, transcription factors and structural proteins. Furthermore, it was observed that oxidative stress increases with the age, with the accumulation of lipid oxidation products, nucleic acids, proteins, sugars and sterols, ultimately causing cellular dysfunctions and making the body more susceptible to harmful external agents.

For the reasons explained above it is clear that it is of fundamental importance, for the evaluation of the health status of a patient and also for the prevention of future diseases, to have available an effective method for evaluation of oxidative stress in a biological sample.

The methods currently available for the evaluation of the ROS species in biological samples, such as blood, plasma, serum, urine etc., are all based on the determination of the oxidation products of the main biological macromolecules, i.e. lipids, proteins, and DNA. Other methods currently available are fluorimetric, colorimetric and luminometric methods, which allow measuring only relative changes of reactive species rather than allowing their quantification in absolute terms.

To date, the method that is considered the "gold standard" for the direct determination of radical species is the electronic paramagnetic resonance (Electron Paramagnetic Resonance, EPR), a spectroscopic technique able to identify and quantify chemical species containing one or more unpaired electrons. However, this technique requires a very sophisticated and expensive equipment and is therefore not suitable for routine laboratory diagnostics.

The need to have available a method and a device, easy to be used and of low cost, which guarantees a reliable assessment of oxidative stress due to the presence of the ROS species, is therefore strongly felt in the field.

### Summary of the Invention

Subject of the present invention is therefore a method for the *in vitro* evaluation of an oxidative stress state associated to ROS and a device for achieving this method, that solves the technical problems highlighted above for the known methods, allowing in particular reducing costs in terms of reagents and equipment required.

A further subject of the present invention is to provide a method and a related device for the *in vitro* evaluation of an oxidative stress state associated to ROS species, which allows implementing accurately quantitative measurements of the oxygen reactive species in a biological sample of a patient, thus providing a reliable result on the oxidative stress grade of the patient her/himself.

These and further subjects are represented by the method for *in vitro* evaluation of the oxidative stress state of a patient and by the related device according to the present invention, whose essential characteristics are defined in the independent claims herein annexed. Further important characteristics of the method and of the device according to the invention are defined in the dependent claims.

### Brief Description of the Drawings

The features and advantages of the method and of the device for evaluation of an oxidative stress state according to the present invention, will be clearly illustrated in the following description as non-limiting exemplary embodiments, also with reference to the annexed figures wherein:
- Figure 1 shows a block diagram that schematically illustrates the structure of a preferred embodiment of the device according to the invention;
- Figure 2 shows, in the form of a histogram, the impedance values measured in blood samples of health patients and of patients under an oxidative stress state by means of the method and of the device of the invention, according to what described in the following Example 1;
- Figure 3 shows, in the form of a histogram, the impedance values measured in blood samples of health patients and of patients under an oxidative stress state by means of the method and of the device of the invention, according to what described in the following Example 2;
- Figure 4 shows a graph obtained by impedance spectroscopy as described in the following Example 3, as a validation of the precision of the invention method.

### Detailed Description of the Invention

Inventors have found a correlation between the impedance values measured in samples of biological fluids of a subject and the amount of markers of oxidative stress dosed in the same fluids, such as in particular oxidised lipids, carbonylated proteins, total antioxidant capacity, ROS levels.

The present invention therefore provides an alternative and more cost-effective, simple and rapid method for determining the degree of oxidative stress in a patient by measuring in particular the impedance values of a patient's blood sample, or of other biological samples. The determination of the degree of alteration of the oxidative state in biological fluids correlated to impedance measurements in the same fluids, which is the basis of the present method, represents a useful tool for the diagnosis, monitoring and prognosis of acute and chronic diseases to which a state of oxidative stress is associated, as well as an interesting and reliable tool to evaluate, more in general, the state of health of a patient.

The present invention also provides a device based on the measurement of impedance values in samples of biological fluids, in particular of blood and derivatives thereof, which allows assessing the oxidative stress state of a patient by testing a sample of the patient's serum in a very simple and rapid way, with measures that have proved to be reproducible and repeatable over time.

A device for evaluation of an oxidative stress state in a subject according to the invention comprises:
- a unit for sampling and for impedance measurement on a biological fluid of the subject under investigation, intended for collecting the fluids samples and for measuring the impedance by means of a couple of electrodes adapted to be immersed in the samples to be tested;
- a processing unit associate to said unit for sampling and impedance measurement is able to collect and process the data coming from said unit through an integrated circuit, to obtain corresponding impedance values of the tested samples, and to send them to a screen for the display of the data,
wherein said unit for sampling and impedance measurement further comprises a DDS frequencies generator (Direct Digital Synthesizer) able to generate signals of frequency in the whole field of investigation of the present method, from 10 Hz to 2MHz, as described in the following.

The processing unit of the present device, once obtained the impedance data related to a sample, can furthermore be configurated for verifying if the measurement value obtained for the impedance of the biological fluid is outside or within a range of reference values measured in a healthy population, so as to establish accordingly if the subject under investigation respectively is or is not in an oxidative stress state, and it provides the so obtained data on a screen or on other data display means.

In the present invention, by "biological sample" or "biological fluid" any biological fluid taken from the subject under investigation is meant, preferably blood and derivatives thereof, such as serum or plasma.

According to the invention, the sampling and measurement unit comprises a container for the fluid sample to be tested and a couple of electrodes able to be immersed in this fluid, which are preferably made of, or anyway coated by, a noble metal, such as in particular gold or platinum, so as to minimize the oxidation processes, that could alter the measurement, and to guarantee the minimum physico-chemical interaction with the biological sample under investigation.

In the present device, the sample of biological fluid to be tested can be advantageously conveyed from the outside to the sampling and measurement unit by an automatic pipette having variable volume. Optionally, before proceeding with the measurement, the sample can be moreover centrifugated and/or filtered, or it can be subjected to another treatment in order to eliminate portions of the sample that may interfere with the measurement.

The processing unit in the present device comprises an integrated circuit that is able to collect and process the electric signals measured in the samples and to send them to a screen.

In a preferred aspect of this invention, the present device, instead of sending the processed data directly to a screen, further comprises a controlling unit comprising a microcontroller, such as an ARM microcontroller (Advanced RISC Machine), suitably programmed so as to manage the transmission and storage of the data processed in a local memory or in a remote station. By "remote station" a personal computer or a smart device is herein meant in particular; according to a preferred embodiment of the invention, it can also generate setting up messages, through the microcontroller, for the sampling and measurement unit and/or for the processing unit.

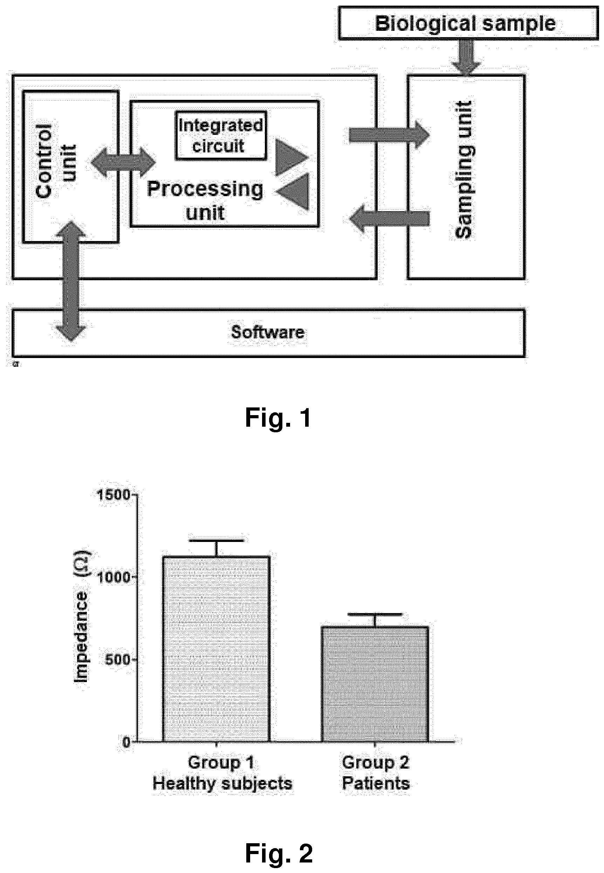

As schematically illustrated in Figure 1, such embodiment of the present device will comprise a software for managing the microcontroller too.

Impedance is in general defined as the total opposition that a circuit, or more in general an object, offers to the alternate current (AC) flow at a given frequency. Many methods are known for measuring impedance; the most appropriate can be selected according to the requirements and the conditions of the measurements, such as the required frequency coverage or the measurement accuracy. The sampling and measuring unit of the present device, and the processing unit connected thereto, can be built with a suitable hardware structure having a software and an interdigitated base plate or a pad strip made from a noble metal such as gold, as the support for the samples to be tested. Suitable examples of a unit for sampling and for measuring impedance and of a connected processing unit are for instance those systems described in "AGILENT IMPEDANCE MEASUREMENT HANDBOOK" 4a edition; or disclosed by Ackman J.J. et al. 1984 Critical Reviews in Biomedical Engineering 11 (4), pp. 281-311; Chen S.W. et al. 2012 Biosensors and Bioelectronics 33(1), pp. 196-203; Dvorskij, V.Ya. et al. 1998 Izvestiya VUZ: Radioelektronika 41 (7), pp. 75-77.

In the present invention the measurements of impedance are typically carried out under an alternate current (AC) regime at a frequency comprised in the range from 10 Hz to 100 kHz with a resolution of at least 1 Hz, so as to minimize the interaction and the polarization effects of those substances still present in blood, serum, or in another biological fluid tested, which could be polarized if subjected to a constant voltage; in this way the properties of the biological fluid under investigation are maintained at the maximum grade.

In the present invention, the measurements of impedance can be advantageously carried out under an alternate current (AC) regime also at a frequency extending up to 2 MHz, therefore in a total range between 10 Hz and 2 MHz. The device of the invention, in order to extend the frequency field up to 2MHz, comprises in the measurement unit a DDS generator suitably designed so as to guarantee an investigation field in the range of frequency 10Hz-2MHz. Such a DDS device (Direct Digital Synthesizer) is suitably connected to a control unit of the device so as to guarantee coherence between the generated signals and the acquired measurement.

The amount of biological fluid typically tested in the present device can vary from some µL to some tens of µL. The impedance values measured for such volumes can vary from 100 Ω to 10000 Ω, with the measuring parameters for achieving such impedance measurements in a frequency range for carrying out a frequency sweeping or at a single frequency depending on the test requirements, with the amplitude of the sample signals that may be set for instance between 200mVpp and 2 Vₚₚ.

The present method for the evaluation of an oxidative stress state in a subject comprises the following steps:
- contacting a sample of a biological fluid previously taken from said subject with a support provided with electrodes;
- measuring impedance of said fluid, obtaining a measured value of impedance;
- verifying if the measured value of impedance for said biological fluid is outside or comprised in a range of reference values measured for a healthy population; and
- assessing accordingly if said subject is respectively in an oxidative stress state or not. By "range of reference values" in the present invention the range of values consisting of mean±2DS (Standard Deviation) is meant, corresponding to the 95% of the values measured in a healthy population.

The experiments carried out by the Applicants and described in the following have shown that the present method and device are efficient in identifying and quantifying the radical species in the blood of the subjects under investigation, or derivatives thereof. Such experiments have in fact proved that the method and device of this invention allow carrying out measurements that are strictly correlated with the ROS concentration in the biological sample examined, highlighting in particular how high values of impedance correspond to low values of ROS in the sample.

The advantages of the present device are first of all represented by the simplicity and rapidity of the measurement, as well as by the inexpensiveness of the method with respect to the known techniques, both in terms of reagents used and in terms of equipment. By the way, in the present method the only treatment done before the impedance measurement can be the preparation of serum or plasma by centrifugation of a sample of the patient's whole blood, and optionally by a filtration, while no reagents nor other particular procedures are required.

A further advantage of the method of the invention is the reliability and reproducibility of the results obtained.

Still a further advantage of the present method is that it is completely reliable and repeatable even if carried out with small volumes of biological fluids, equal to about 15-20 µL, without the need of taking from the patient under investigation high amounts of blood or of other fluids.

Finally, a further advantage of this invention is that the device of the invention can be made of small dimensions, with disposable parts, and it can be automatable.

The device and related method of the invention, as illustrated above, shall be used for applications in the diagnosis/prognosis/therapy of several diseases in acute or chronic forms, but also as a means of diagnosis or as a research tool in assisted reproduction techniques, sports medicine, nutrition, aging, and in any other field wherein the role of oxidative stress was widely proved in the scientific literature.

### EXPERIMENTAL PART

### Example 1

Impedance measurements were performed with the device of the invention at 30 kHz on blood samples in drops of the volume of 20 µL each from two groups of subjects. A first group consisted of 40 subjects with signs of systemic oxidative stress caused by ongoing inflammatory diseases and evidenced by the decrease in total antioxidant capacity and increase in lipid peroxidation (Total antioxidant capacity: 15.2 ± 2.8 mM equivalents of Trolox®; Peroxidation lipid: 1.12 ± 0.56 µM of malondialdehyde (MDA)). A second group, used as a control, consisted of 40 healthy subjects (without alterations of the systemic redox status shown by: total antioxidant capacity: 23.6 ± 2.4 mM equivalents of Trolox® and lipid peroxidation: 0.41 ± 0.21 µM of MDA.

The impedance values measured with the device and method of this invention, shown in Figure 2 in the form of a histogram for the two groups of subjects (healthy controls: 1123 ± 97 Ω; subjects with systemic oxidative stress: 682 ± 84 Ω), resulted as significantly correlated (p <0.05), the examined levels of oxidative stress markers, i.e. oxidized lipids (r = 0.66) and total antioxidant capacity (r = 0.80), being measured by photometric / fluorimetric methods.

### Example 2

The following tests were conducted on 10 blood samples of healthy subjects (without alterations in the systemic redox status).

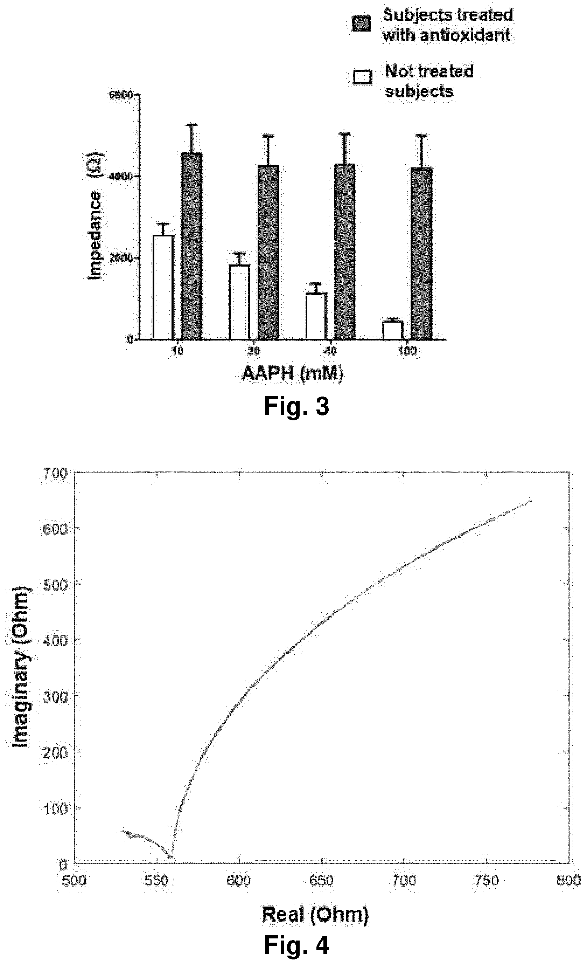

Test A: increasing concentrations of 2,2'-azobis (2-amidinopropane) dihydrochloride (in the following "AAPH"), an azo-compound known for its ability to generate radical species, have been added to the aforementioned blood samples, then measuring the impedance with the device of this invention. As shown in Figure 3, in the white histograms, as the concentration of the added AAPH increased, a consequent decrease in impedance values measured at 30 kHz was observed (10 mM AAPH: 2544 ± 300 Ω; 20 mM AAPH: 1823 ± 287 Ω; 40 mM AAPH: 1124 ± 241 Ω; 100 mM AAPH: 432 ± 88 Ω).

Test B: The same experiment was conducted in the presence of the antioxidant Trolox®, added to the samples in such quantities as to obtain a final concentration of 40 µM, a water-soluble analogue of Vitamin E. In the presence of this antioxidant a significant increase in impedance value, as shown in Figure 3, histograms in black (10 mM AAPH: 4577 ± 688 Ω; 20 mM AAPH: 4255 ± 741 Ω; 40 mM AAPH: 4288 ± 755 Ω; 100 mM AAPH: 4188 ± 822 Ω).

These experiments allowed verifying that low concentrations of free radicals in the samples correspond to high impedance values, and that the two parameters are strictly correlated.

### Example 3

Impedance measurements were performed with the device of the invention, in the frequency range10Hz-2MHz, on 20 µL of a blood sample of a same subject. In particular, the sample was measured 5 consecutive times. In the graph in Figure 4, in which each curve indicates a different measure, a concordance between the measurements repeated on the same sample can be appreciated and therefore the high analytical precision of the measurement method of this invention.

The present invention has been described above with reference to preferred embodiments thereof, but further preferred embodiments may exist, all included in the same inventive core, as defined by the scope of the annexed claims.

## Claims

1. A method for the assessment of an oxidative stress state in a subject, comprising the following steps:
- contacting a sample of a biological fluid previously taken from said subject with a support provided with electrodes;
- measuring the impedance of said fluid, obtaining a measured value of impedance;
- verifying if the measured value of impedance for said biological fluid is outside or comprised in a range of reference values of impedance measured for a healthy population; and
- assessing accordingly if said subject is respectively in an oxidative stress state or not.

2. The method according to claim 1, wherein said support provided with electrodes is a support made of or coated by a noble metal forming a couple of electrodes.

3. The method according to claim 1 or 2, wherein noble metal is selected from between gold and platinum.

4. The method according to any one of the preceding claims, wherein said measuring of impedance is carried out in alternate current (AC) regime at a frequency ranging from 10 Hz to 2MHz.

5. A device configured for the assessment of an oxidative stress state of a subject by measurement of the impedance of a biological fluid sample of said subject, said device comprising:
- a sampling and measurement unit comprising a container for a sample of a biological fluid of said subject, a couple of electrodes adapted to be immerged in said sample, and further comprising a DDS (Direct Digital Synthesizer) frequency generator adapted to generate frequency signals in the range between 10 Hz and 2MHz;
- a processing unit associated to said sampling and measurement unit adapted for collecting and processing the data coming from said sampling and measurement unit through an integrated circuit, to obtain corresponding impedance values of the samples tested and to verify if said values are outside or comprised in a range of reference values, and for sending them to a screen for the display of data.

6. The device according to claim 5, further comprising a centrifuge and a filtering device in said container for the obtainment of serum from whole blood when said sample of biological fluid consists of whole blood.

7. The device according to any one of claims 5 or 6, further comprising an automatic pipette having a variable volume for conveying said sample of biological fluid from outside into said sampling and measurement unit.

8. The device according to any one of claims 5-7, wherein said couple of electrodes is made of or it is coated by a noble metal selected from between gold and platinum.

9. The device according to any one of claims 5-8, further comprising a control unit comprising a microcontroller able to manage the transmission and storage of data, processed by said processing unit, in a local storage system or in a remote station.
